# EUROPEAN PATENT APPLICATION

(11) **EP 2 161 578 A1**
(43) Date of publication of application: **10.03.2010**
(21) Application number: 08168320.3
(22) Date of filing: 29.05.2002
(51) Int. Cl.: G01N 33/68, C12Q 1/68, C07K 16/00, A61K 38/17, C07K 14/47

(54) **Proteins in diabetes proteome analysis**

(30) Priority: 29.05.2001 DK 200100852
(62) Divisional of application: 02742845.7
(71) Applicant: Pride Proteomics A/S, 5230 Odense M (DK)
(72) Inventor: Larsen, Peter Mose, 5260 Odense S (DK); Fey, Stephen, 5491 Blommeslyst (DK); Nerup, Jörn, 2840 Holte (DK); Karlsen, Allan, 3450 Allerod (DK); Nielsen, Karin, 4600 Koege (DK)
(74) Representative: Orsnes, Henrik Egede

(57) **Abstract**

Provided is a method for diagnosing diabetes in a human, wherein the method comprises establishing in a biological sample from the human an increased expression of marker protein Zinc Finger protein 43 (P28160). Moreover, there is provided a test kit for diagnosing diabetes or a genetic predisposition for diabetes in a mammal.

## Description

### FIELD OF INVENTION

Proteome analysis has allowed for the identification of selected proteins associated to diabetes. Thus, these proteins, in themselves, either up-regulated or down-regulated, are indicators of diabetes in a patient. The pattern of regulation of a group of these proteins also serves as an indicator of diabetes. These proteins can be used as targets for the treatment of diabetes or for treatment itself. The proteins were identified by monitoring changes in protein expression during β-cell maturation.

### BACKGROUND OF THE INVENTION

Development of Type 1 Diabetes Mellitus (T1DM) is characterized by mononuclear cell infiltration in the islets of Langerhans (insulitis) and selective destruction of the insulin producing β-cells [1, 2]. It is generally accepted that the autoimmune destruction of the β-cells results from interactions between various environmental factors and immune mechanisms in genetically susceptible individuals [3]. The very first events initiating the destructive process have not been described yet. Cytokines, in particular intedeukin-1β (IL-1β), are known to be released within the islets in low concentrations by a limited number of nonendocrine cells in sufficient quantities to inhibit and modulate the β-cell function in vitro [4]. In response to low concentrations of IL-1β islets increase insulin release but insulin release is decreased at high concentrations. Furthermore IL-1β influences many important cellular functions such as decreasing DNA synthesis, decreasing protein synthesis and intracellular energy production and induction of apoptosis. Many of these effects are mediated through induction of the inducible NO syntase (iNOS) and its product, the free radical nitric oxide (NO•) [5]. The present investigators hypothesize that the β-cell when exposed to IL-1β initiates a self protective response in competition with a series of deleterious events, and that in β-cells the deleterious prevail [3]. In support of this, overexpression of scavengers of free radicals such as catalase and glutatione peroxidase reduces the deleterious effects of cytokines on β-cells [6].

During development of the pancreas, all four endocrine cell-types, (the insulin producing β-cells, the glucagon producing α-cells, the somatostatin producing δ-cells and the pancreatic polypeptide producing PP-cells) are generally believed to arise from the same stem cell [Pictet, 1972]. This is further supported by the demonstration of double positive glucagon and insulin producing cells during the early stages of β-cell development. However, later in the maturation process the double positive stem cells maturate into two distinct cell-types producing either glucagon or insulin [Alpert, 1988; Hashimoto, 1988]. β-cell specific sensitivity to cytokines and free radicals, may therefore represent an acquired trait during maturation of the stem-cells into mature insulin producing β-cells, since sensitivity to cytokines is not found in the other endocrine cell-types. In all cells expressing cytokine-receptors, both protective and deleterious processes are induced by cytokines.

### SUMMARY OF THE INVENTION

The high-resolution proteome technology effectively separates and identifies proteins with high success rate. Compared to analyses of mRNA expression, proteome analysis offers the possibility of relative quantification of changes in protein expression as well as identification of post-translattion protein modifications such as phosphorylation, methylation and cleavage. Post-translational modifications are often required for the functional activation of a protein and hence, may be of pathogenenic importance. The present investigators illustrate this point herein wherein at least 24 percent of the identified proteins reflect post-translational modification.

In investigating whether changes in the protein expression pattern increasing the sensitivity to cytokines is a consequence of β-cell maturation, the present investigators used two different cell-types: a glucagon producing pre-β-cells (NHI-glu), which maturate to an insulin producing β-cellos (NHI-ins). Without being bound to a particular theory, the maturation process of these two phenotypes is believed to resemble separate stages in the maturation of the α-cell phenotype and the β-cell phenotype during normal islet cell development. Previous analyses of these two phenotypes by the present investigators demonstrated that this maturation process was accompanied with acquired sensitivity to the toxic effect of high concentrations of IL-1β [Nielsen, 1999].

For this purpose, proteome analysis is a useful approach. The present investigators previously used proteome analysis to identify changes in rat and human islet protein expression in response to cytokines. Here, proteome analysis is used to identify changes in the protein expression profile accompanying maturation of the IL-1β sensitive β-cell phenotype.

A first aspect of the invention relates to a method for diagnosing diabetes in a human. The method comprises determining the presence or level of expression of at least one marker protein in a biological sample from the human, wherein the marker protein is selected from the group consisting of

**(Table 1)**

| **Gel spot no:** | **Protein** | **Database Acc #** | **MW** | **Theor MW** | **pl** | **Theor pl** |
|---|---|---|---|---|---|---|
| NEPHGE 76 | Phosphoglycerate kinase * | P16617 | 37.1 | 44.4 | 8.3 | 7.5 |
| NEPHGE 124, 193, 241, 105# | Fructose-bisphosphate aldolase A * | P05065 | 20.7, 35.4, 34.9, 35.6 | 39.2 | 8.6, 8.9, 8.9, 8.3 | 8.4 |
| NEPHGE 568 | Glyceraldehyde-3-phophate-dehydrogenase * # | P04797 | 35.7 | 35.7 | 7.8 | 8.4 |
| IEF 166* | Enolase α | P04764 | 49.6 | 47.0 | 5.7 | 6.2 |
| IEF 193, 1219 | Enolase γ* | P07323 | 47.8, 62.8 | 47.0 | 5.0, 5.1 | 5.0 |
| IEF 255* | Transaldolase | Q93092 | 37.7 | 37.4 | 6.1 | 6.6 |
| IEF 794 | Glyceraldehyde-3-phophate-dehydrogenase | M17701 | 35.9 | 35.7 | 6.8 | 8.4 |
| IEF 1472*, 1473* | Puruvate kinase, M1 isozyme | P11980 | 53.5, 53.5 | 57.7 | 7.2, 7.1 | 6.7 |
| NEPHGE 77* | Argininosuccinate synthase | P00966 | 40.1 | 46.4 | 8.1 | 8.4 |
| NEPHGE 105# | Heterogeneous nuclear ribonucleoprotein A3 | P51991 | 35.6 | 39.7 | 8.3 | 8.7 |
| NEPHGE 105# | Poly (RC) binding protein 2 | Q61990 | 35.6 | 38.2 | 8.3 | 6.3 |
| NEPHGE 230* | EIF-2-gamma Y | Q9Z0N2 | 42.6 | 51.0 | 8.8 | 8.8 |
| NEPHGE 357# | 40S ribisomal protein S25 | P25111 | 20.1 | 13.7 | 7.7 | 10.1 |
| NEPHGE 357# | 40S ribosomal protein S18 | P25232 | 20.1 | 17.7 | 7.7 | 11.0 |
| NEPHGE 551 | Heterogeneous nuclear ribonucleoprotein A2/B1 # | P22626 | 35.1 | 37.4 | 9.0 | 9.0 |
| NEPHGE 4410* | 60S ribisomal protein L26 | P12746 | 21.0 | 17.3 | 8.0 | 10.6 |
| NEPHGE 4410* | Ubiquitin-conjucating enzyme E2 | 076069 | 21.0 | 20.9 | 8.0 | 7.6 |
| IEF 255* | 60S Acidic ribisomal protein P0 | P19945 | 37.7 | 34.2 | 6.1 | 5.9 |
| IEF 256 | Pyridoxal kinase | 035331 | 39.1 | 34.9 | 6.3 | 6.3 |
| IEF 383* | Isbvaleryl-CoA dehydrogenase | P12007 | 46.4 | 46.4 | 6.2 | 8.0 |
| IEF 383 | Ubiquitin fusion degradetion protein 1 homolog | P70362 | 46.4 | 34.5 | 6.2 | 7.0 |
| IEF 403, 1039*, 1500* | 26S protease regulatory subunit 7 | Q63347 | 48.6, 36.8, 39.5 | 48.6 | 5.6, 5.8, 5.8 | 5.6 |
| IEF 12315* | Translation initiation factor 5 | Q07205 | 77.7 | 49.0 | 4.8 | 5.4 |
| NEPHGE 14 | Isocitrate dehydrogenase | P54071 | 39.8 | 58.7 | 8.6 | 8.9 |
| NEPHGE 77* | Citrate synthase | 075390 | 40.1 | 51.7 | 8.1 | 8.1 |
| NEPHGE 252, 4234* | Voltage-dependent anion-selective channel protein 1 | Q60932 | 30.6, 30.5 | 30.6 | 8.0, 8.0 | 8.6 |
| NEPHGE 335* | Phosphoenolpyruvate carboxylase | P29195 | 22.4 | 109.4 | 7.6 | 5.8 |
| NEPHGE 377,516 | ATP synthase alpha chain # | P15999 | 56.8, 51.6 | 58.8 | 8.0, 8.1 | 9.2 |
| NEPHGE 582, 4234*, 45124 | Voltage-dependent anion-selective channel protein 2 # | P81155 | 31.6, 30.5, 32.6 | 31.7 | 7.4, 8.0, 7.9 | 7.4 |
| NEPHGE 20140 | Fumarate hydratase | P14408 | 42.9 | 54.5 | 8.1 | 9.1 |
| IEF 123 | cAMP-depend. protein kinase type I-alpha regu. chain | P09456 | 48.3 | 43.0 | 5.3 | 5.3 |
| IEF 359 | Isocitrate dehydrogenase | P41562 | 48.7 | 46.7 | 6.5 | 6.5 |
| IEF 616* | Creatine kinase, B chain # | P07335 | 43.5 | 42.7 | 5.3 | 5.3 |
| IEF 700, 1296 | G25 GTP-binding protein | P25763 | 21.3, 23.4 | 21.3 | 6.1, 6.2 | 6.2 |
| NEPHGE 59* | RAN | P17080 | 24.9 | 37.8 | 8.0 | 9.4 |
| NEPHGE 156*, 303* | T-complex protein 1, beta subunit | Q99832 | 47.5, 47.7 | 59.4 | 8.4,8.3 | 7.6 |
| NEPHGE 332 | RAS-related protein RAB-5C | P51148 | 23.6 | 23.6 | 8.3 | 8.9 |
| NEPHGE 453 | Peptidyl-prolyl cis-trans isomerase A | P10111 | 17.7 | 17.7 | 8.4 | 8.4 |
| IEF 82, 85*, 1463* | Hsc 70-ps1 | CAA496 70 | 61.9, 72.3, 62.0 | 70.9 | 5.4, 5.1, 5.4 | 5.4 |
| IEF 85*, 775*, 846*, 1358 | 78 Kda glucose-related protein * # | P06761 | 72.3, 70.0, 40.5, 96.0 | 72.3 | 5.1, 5.1, 6.1, 4.9 | 5.1 |
| IEF 109*, 542, 806, 973* | Probable protein disulfide isomerase ER-60 * # | P11598 | 54.7, 59.5, 24.1, 58.3 | 56.6 | 5.6, 5.7, 4.6, 6.3 | 5.9 |
| IEF 109* | T-complex protein 1, theta sububit | P42932 | 54.7 | 59.6 | 5.6 | 5.4 |
| IEF151 | ERJ3 protein | Q9UBS4 | 49.7 | 40.5 | 6.1 | 5.8 |
| IEF 376 | N-ethylmaleimide sensitive factor | Q9QUL6 | 65.2 | 82.7 | 6.4 | 6.6 |
| IEF 408 | Clatrin light chain | AAA40891 | 59.6 | 25.1 | 4.6 | 4.6 |
| IEF 463 | RAS-related protein RAB-11 B | P46638 | 25.2 | 24.5 | 6.3 | 5.6 |
| IEF 469#, 1472*, 1473* | T-complex protein, zeta subunit* | P80317 | 59.7, 53.5, 53.5 | 58.0 | 6.3, 7.2, 7.1 | 6.6 |
| IEF 583 | Vesicular-fusion protein NSF | P46460 | 64.5 | 82.6 | 6.4 | 6.5 |
| IEF 728^, 881# | T-complex protein 1, gamma subunit | P49368 | 67.4, 62.9 | 60.3 | 6.0, 6.2 | 6.2 |
| IEF 728^, 469#, 881#, NEP 282 | P60 protein | 035814 | 67.4, 59.7, 62.9, 61.1 | 62.6 | 6.0, 6.3, 6.2, 7.4 | 6.4 |
| IEF 730 | Hsc 70-interacting protein | P50503 | 49.5 | 41.3 | 5.1 | 5.3 |
| IEF 871*, 728^ | Coatomer delta subunit (bovin, human)* | P53619 | 70.1, 67.4 | 57.2 | 6.0, 6.0 | 5.9 |
| IEF 922 | Kinesin heavy chain | P33176 | 92.1 | 109.9 | 5.9 | 6.1 |
| IEF 1014* | Amphiphysin-like protein | 008839 | 84.2 | 64.5 | 5.0 | 5.0 |
| IEF 1039*, 1500* | Sorting Nexin 6 | Q9UNH7 | 36.8, 39.5 | 46.6 | 5.8, 5.8 | 5.8 |
| IEF 1451* | Apolipoprotein A-I | P02647 | 58.2 | 30.8 | 6.9 | 5.6 |
| IEF 1463* | Mortalin (GRP75)* | P48721 | 62.0 | 73.9 | 5.4 | 6.0 |
| IEF 1513 | Alpha-soluble NSF attachment protein | P54921 | 16.0 | 33.2 | 6.0 | 5.3 |
| IEF 9224 | Heat-shock protein 105 Kda | Q61699 | 87.6 | 96.5 | 5.5 | 5.4 |
| NEPHGE 356 | Transgelin 2 | P37802 | 22.4 | 22.4 | 8.2 | 8.4 |
| NEPHGE 447 | Neurofilament triplet H protein | P16884 | 21.3 | 89.5 | 7.9 | 5.6 |
| NEPHGE 454 | Complement component C4 | Q29439 | 18.0 | 14.5 | 8.3 | 5.3 |
| NEPHGE 454 | Destrin | JE0223 | 18.0 | 18,4 | 8.3 | 7.8 |
| NEPHGE 19991 | Caldesmon | Q05682 | 58.8 | 93.3 | 8.1 | 5.6 |
| IEF 104, 612, 616* | Keratin, type II cytoskeletal 8 # | Q10758 | 53.9, 45.3, 43.5 | 53.9 | 5.7, 5.5, 5.3 | 5.8 |
| IEF 202,1193 | Alpha-2-macroglobulin receptor-associated protein | Q99068 | 43.5, 45.4 | 41.7 | 6.5, 6.7 | 6.9 |
| IEF 215 | Serine/threonine protein phosphatase PP1-beta | P37140 | 37.2 | 37.2 | 6.2 | 5.8 |
| IEF 232 | PKCq-interacting protein PICOT | AAF28843 | 40.9 | 31.4 | 5.8 | 4.9 |
| IEF 330* | Cofilin, non-muscle isoform | P45592 | 18.3 | 18.5 | 6.5 | 8.2 |
| IEF 469# | Dihydropyrimidase related protein-1 (CRMP-1) | Q62950 | 59.7 | 62.2 | 6.3 | 6.6 |
| IEF 565, 12315*, 12340 | Protein disulfide isomerase | P04785 | 86.6, 77.7, 116.3 | 57.0 | 4.9, 4.8, 5.0 | 4.8 |
| IEF 604, 1438 | Ezrin | P26040 | 76.2, 81.0 | 69.2 | 6.0, 5.8 | 5.8 |
| IEF 662 | Nonmuscle myosin heavy chain-B | AAF61445 | 94.4 | 22.9 | 5.7 | 5.5 |
| IEF 900 | Reticulocalbin 1 | Q05186 | 105.7 | 38.1 | 4.7 | 4.7 |
| IEF 728^, 871*, 881# | Turned on after division 64 (TOAD 64) (CRMP-3) * # | P47942 | 67.4, 70.1, 62.9 | 62.3 | 6.0, 6.0, 6.2 | 6.0 |
| IEF 935, 1014* | Endoplasmin | P08113 | 98.5, 84.5 | 92.5 | 4.7, 5.0 | 4.7 |
| IEF 973*, 1351 | Lamin A* | P48679 | 58.3, 66.0 | 74.3 | 6.3, 6.2 | 6.5 |
| IEF 1020 | Myosin heavy chain | Q90337 | 72.4 | 221.1 | 4.6 | 5.6 |
| IEF 1154 | Lamin B1 | P70615 | 68.4 | 66.6 | 5.2 | 5.2 |
| IEF 1451* | Fibrinogen gamma-a chain | P02679 | 58.2 | 49.5 | 6.9 | 5.6 |
| IEF 1482 | Vitamin D-binding | P04276 | 46.6 | 53.5 | 5.5 | 5.7 |
| IEF 1564 | Fatty acid-binding protein, epidermal | P55053 | 11.6 | 15.1 | 6.3 | 6.7 |
| NEPHGE 59* | PAX 1 | P15863 | 24.9 | 24.4 | 8.0 | 6.6 |
| NEPHGE 156* | Lamina-associated poypeptide 2 | Q62733 | 47.5 | 50.3 | 8.4 | 9.4 |
| NEPHGE 230*, 20127# | Flag structure-specific endonuclease | AAF81265 | 42.6, 41.2 | 42.6 | 8.8, 8.6 | 8.8 |
| NEPHGE 357# | RNA polymerase II transscriptional coactivator P15 | Q63396 | 20.1 | 13.7 | 7.7 | 9.7 |
| NEPHGE 458 | Lupus la protein homolog | P38656 | 19.4 | 47.8 | 8.0 | 9.7 |
| NEPHGE 526 | DNA-polymerase | P78988 | 42.9 | 99.5 | 9.4 | 8.9 |
| NEPHGE 19980, 45036 | Septin-like protein | Q9QZR6 | 54.5, 64.4 | 63.8 | 8.4, 7.8 | 8.7 |
| NEPHGE 20127# | Hypothetical 44.7 protein | CAB664 81 | 41.2 | 44.7 | 8.6 | 7.6 |
| NEPHGE 20127# | CDC10 protein homolog | Q9WVC0 | 41.2 | 50.5 | 8.6 | 8.8 |
| IEF 156 | NEDD 5 protein | P42208 | 38.0 | 41.5 | 6.1 | 6.1 |
| IEF 166* | Histidyl-tRNA synthetase | Q61035 | 49.6 | 57.4 | 5.7 | 5.7 |
| IEF 313 | Zinc Finger protein 43 | P28160 | 27.2 | 93.5 | 6.4 | 9.4 |
| IEF 330* | Nucleoside diphophate kinase B | P19804 | 18.3 | 17.3 | 6.5 | 6.9 |
| IEF 462 | Cytidylate kinase # | P30085 | 23.3 | 22.2 | 6.3 | 5.4 |
| IEF 775* | Heterogeneous nuclear ribonucleoprotein K | Q07244 | 70.0 | 51.0 | 5.1 | 5.4 |
| IEF 846* | Zinc finger protein 26 | P10076 | 40.5 | 48.9 | 6.0 | 9.3 |
| IEF 850* | Reverse transcriptase | Q9YQW2 | 57.8 | 27.9 | 6.1 | 9.3 |
| IEF 885 | Importin alpha | Q9Z0N9 | 48.5 | 57.8 | 5.4 | 5.4 |
| IEF 1209 | FUSE binding protein 2 | Q92945 | 71.7 | 68.4 | 6.5 | 8.5 |
| IEF 5223 | Dynactin, 50 Kda isoform | Q13561 | 50.2 | 44.8 | 5.1 | 5.1 |
| NEPHGE 303* | Coding region determinant binding protein | 088477 | 47.7 | 63.5 | 8.3 | 9.3 |
| NEPHGE 335* | Polyubiquitin | Q63654 | 22.4 | 11.2 | 7.6 | 5.4 |
| NEPHGE 441 | Glutathione S-transferase P | P46524 | 23.5 | 23.5 | 8.0 | 8.1 |
| NEPHGE 605 | Glutathione S-transferase YB1 | P04905 | 25.9 | 25.9 | 8.8 | 8.4 |
| IEF 482 | Neurolysin | P42676 | 80.3 | 80.3 | 5.6 | 6.0 |
| IEF 850* | Proteasome component C2 | P18420 | 57.8 | 29.5 | 6.1 | 6.1 |
| IEF 1508 | Arginase 1 | P07824 | 43.1 | 35.0 | 6.8 | 6.8 |

and marker proteins further consisting of modifications and derivatives of marker proteins of Table 1, so as to have at least 80% homology with marker proteins of Table
1,
wherein pI is the isoelectric point of the marker protein as determined by isoelectric focusing, and the molecular weight (MW) is determined on a polyacrylamide gel.

The invention further relates to a method of treating diabetes in a human comprising administering a marker protein of Table 1, a nucleotide sequence coding for a marker protein of Table 1, an antibody for a protein of Table 1, a nucleic acid fragment capable of binding to a marker protein of Table 1, or a compound capable of binding to a marker protein of Table 1 to said human.

An interesting object of the invention relates to a method for determining the predisposition in a human for diabetes, the method comprising determining the presence or relative level in a biological sample from the human of at least one marker protein
wherein the marker protein being indicative of a predisposition for having diabetes is selected from the group consisting of (Table 1) and marker proteins further consisting of modifications and derivatives of marker proteins of Table 1, so as to have at least 80% homology with marker proteins of Table 1, wherein pI is the isoelectric point of the marker protein as determined by isoelectric focusing, and the molecular weight (MW) is determined on a polyacrylamide gel.

A further object of the invention is to provide a method for diagnosing the predisposition in a human for diabetes, the method comprising
i) establishing the increased expression in a biological sample from the human of at least one marker protein from a biological sample from the human, said marker protein selected from the group consisting of proteins of Table 2;
   or comprising
ii) establishing the decreased expression of at least one marker protein down-regulated marker protein in a biological sample from the human said marker protein selected from the group consisting of proteins of Table 1.
or combinations of steps i) and ii)

An interesting object of the invention relates to a method of preventing or delaying the onset or of diabetes in a human comprising administering a marker protein of Table 1, a nucleotide sequence coding for a marker protein of Table 1, an antibody for a protein of Table 1, a nucleic acid fragment capable of binding to a marker protein of Table 1, or a compound capable of binding to a marker protein of Table 1 to said human.

A further object of the invention is to provide for a method of determining the likelihood of an agent having a therapeutic effect in the treatment of diabetes comprising determining the level of expression of one or more proteins of Table 1 before and after exposing a test model to said agent and comparing said levels. Similarly, the invention relates to a method of determining the effect of a compound in the treatment of diabetes comprising determining the level of expression of proteins of one or more proteins of Table 1 and to a method of determining the level of effect of a compound used in the treatment of diabetes comprising determining the level of expression of one or more proteins of Table 1 before and after exposing a test model to said agent. The present contribution to the art allows for a method of determining the nature or cause of diabetes in a human having or susceptible to said disease comprising establishing the level of expression of a protein of Table 1 in relation to a model.

A further aspect of the invention is directed to a nucleic acid fragment comprising a nucleotide sequence (whether DNA, RNA, LNA or other substituted nucleic acid) which codes for a peptide defined in Table 1.

An antibody including antiomere, hybrid molecules, peptides, ligands and other synthetic molecules hybrid molecule, peptides ligands and other synthetic molecules able to bind to a protein defined in Table 1 is anticipated by the present invention as well as the use of such an antibody for detecting the presence of a peptide defined in Table 1.

A particularly interesting aspect of the invention relates to a test kit for diagnosing diabetes or a genetic predisposition for diabetes in a mammal, comprising:
a) a binding mean which specifically binds to at least one marker protein shown in Table 1 or an antibody for a protein of Table 1, a nucleic acid fragment capable of binding to a marker protein of Table 1, or a compound capable of binding to a marker protein of Table 1 to said human,
b) means for detecting binding, if any, or the level of binding, of the binding means to at least one of the marker proteins or at least one of the peptides or at least one of the nucleic acid fragments (where the nucleic acid is DNA, RNA, LNA or other substituted nucleic acid), and
c) means for correlating whether binding, if any, or the level of binding, to said binding means is indicative of the individual mammal having a significantly higher likelihood of having diabetes or a genetic predisposition for having diabetes.

The invention further relates to a method for determining the effect of a substance, the method comprising using a mammal which has been established to be an individual having a high likelihood of having diabetes or a genetic predisposition for having diabetes by use of the method of claim 1, the method comprising administering the substance to the individual and determining the effect of the substance.

A pharmaceutical composition which comprises a substance which is capable of regulating the expression of a nucleic acid fragment coding for at least part of a protein of Table 1, or at least one marker protein in Table 1, an antibody, including antiomere, hybrid molecules, peptides, ligands and other synthetic molecules hybrid molecule, peptides ligands and other synthetic molecules for a protein of Table 1, a nucleic acid fragment (whether DNA, RNA, LNA or other substituted nucleic acid) capable of binding to a marker protein of Table 1, or a compound capable of binding to a marker protein of Table 1 to said human is further anticipated by the present investigators.

The invention also relates to the use of a gene expressing a protein of table 1 in a method for manufacturing an artificial or improved beta-cell, such as a cell for transplantation into a human, and the engineered cells as such. Specifically, the invention relates to a method for engineering self-cells into beta-cells (sensing glucose and secreting insulin) or improving existing or new "beta-cell lines" or other cell -lines in that direction. In addition, the beta-cells can be made more resistant to an immunological attack by the immunesystem, e.g. more resistant to cytokines. The cell is useful for drug testing or treatment by introduction into a person suffering from diabetes, and can be a part of a pharmaceutical composition.

### GENERAL DESCRIPTION OF THE INVENTION

Proteome analysis has allowed for the identification of proteins and their association to diabetes. These proteins, in themselves, either up-regulated or down-regulated, are indicators of diabetes in a patient. The pattern of regulation of a grouping of these proteins also serves as an indicator of diabetes. These proteins can be used as targets for the treatment of diabetes or for treatment itself. The proteins were identified monitoring changes in protein expression during β-cell maturation.

Maturation of the β-cells in the pancreas is a complex and still unknown mechanism. Many different proteins are involved and their specific functional importance for β-cell maturation needs to be elucidated.

By using mass spectrometry, 108 protein-spots were positively identified given rise to 109 different proteins (Table 1), whereas neither positive protein identification, nor useful mass spectra could be obtained for 27 of the altered protein-spots. The present investigators have found that at least 24 % of the observed changes in protein expression levels may reflect post-translational modification, since 15 proteins were present in two protein-spots (gamma enolase, pyrvuvate kinase M1 isozyme, voltage-dependent anion-selective channel protein 1, ATP synthase alpha chain, G25 GTP-binding protein, septin-like protein, T-complex protein 1 eta and gamma, coatomer delta subunit, sorting nexin 6, flag structure-specific endonuclease, alpha-2-macroglobulin receptor-associated protein, endoplasmin, lamin A and ezrin), 7 proteins were present in three spots (26S protease regulatory subunit, voltage-dependent anion-selective channel protein 2, Hsc70-ps1, T-complex protein 1 zeta, protein disulfide isomerase, keratin type II cytoskeletal 8 and turned on after division 64 (TOAD 64)) and 4 proteins were present in four spots (fructose-bisphosphate aldolase A, 78 Kda glucose regulated protein, probable protein disulfide isomerase ER-60 and P60 protein). Some protein-spots contained more than one protein; 23 protein-spots contained two identified proteins (NEPHGE 59, 77, 156, 230, 303, 335, 454, 4234, 4410 and IEF 85, 109, 166, 255, 330, 383, 616, 775, 846, 850, 871, 973, 1014, 1039, 1451, 1463, 1472, 1473, 1500 and 12315), 5 protein-spots (NEHPGE 105, 357, 20127 and IEF 469 and 881) contained three identified proteins and 1 protein-spot (IEF 728) contained four identified proteins.

Only minor inconsistencies were found between observed and theoretical calculated MW. These inconsistencies may reflect post-translational modifications or could be due to relatively imprecise MW determination at the edge of the gels (NEPHGE 335 and IEF 408, 900, 1020, 1451, 12315). In contrast, no significant differences between observed and theoretical calculated pI values were detected.

The identified proteins have been assigned in 6 groups according to their major known or putative functions: 1) glycolytic enzymes, 2) aminoacid pathway and protein synthesis/degradation, 3) energy transduction, 4) cytokinesis, nucleoacid synthesis, transcription and nuclear transport, 5) chaperones, translocation, protein folding and cellular transport and 6) signal transduction, regulation, growth, differentiation and apoptosis. The function and possible importance for β-cell maturation and T1DM pathogenesis are discussed below for selected proteins.

Thus, the present investigators have identified proteins associated with diabetes by detecting the absolute or relative presence of the proteins of Table 1 in a biological sample. Typically, the biological sample is selected from the group consisting of urine, blood, lymphatic fluids, secretions into the duodenum and tissue. Suitably, the tissue is pancreatic tissue.

Much research has been done to characterize the molecular mechanisms involved during the development of the pancreas. It is generally believed that the endocrine, exocrine and ductal cell-types are derived from endodermal cells [Pictet, 1972, Le Douarin, 1888] Sander1997, St-Onge 1997). But the early steps that control the development of the pancreas and later the mechanisms that specify the different pancreatic cell-types are, however, not well understood. Some analysis is based on the knowledge that during the development of the pancreas co-appearance of different islet hormones is first expressed in mixed phenotypes and later in mature single-hormone-expressing phenotypes [Alpert, 1988; De Krijger, 1992; Teitelman, 1993; Guz, 1995]. However, several additional studies have demonstrated that the pro-endocrine cells require different transcription factors (PDX-1, Nkx-2.2, beta-2/Neuro-D, GLUT-2, Pax4 and Pax6) to mature into the single-expression phenotypes, but it is still under discussion whether co-expressing cells occur during the maturation of the specific cell-types in the pancreas.

The NHI-cell-system is a unique cell-system to analyze mechanisms involved during β-cell maturation accompanied with acquired sensitivity to the toxic effect of IL-1β, since the NHI-ins phenotype is sensitive to IL-1β compared to the NHI-glu phenotype [Nielsen, 1999]. Despite the difference in the sensitivity between the two phenotypes the glucagon-producing NHI-glu phenotype is closely related to the β-cell phenotype according to the mRNA expression profile [Jensen, 1996]. This means that it is necessary to look for a small difference in the protein pattern that makes the NHI-ins phenotype sensitive to the toxic effect of IL-1β in contrast to NHI-glu phenotype.

According to the data presented here a magnitude of protein expression changes is observed during maturation from the pre-β-cell to the β-cell phenotype. Some of these proteins may be of importance for the development of the native mature β-cell and others for the acquired sensitivity to cytokines. In table 1 the proteins are grouped according to their known or putative functions. It is not possible to discuss in detail all the identified proteins, but a few selected proteins, which may be relevant for β-cell maturation or β-cell destruction is discussed below.

Many different genes have been demonstrated to be involved in β-cell maturation, such as pancreatic duodenal homeobox 1 gene (Pdx-1) [Madsen, 1997; Offield, 1996], members of the notch [Lammert, 2000; Jensen, 2000] and paired-homeodomain box (Pax) family. In the Pax family the transcription factors Pax-8/9 have been found in the foregut, which give rise to thyroid follicle cells [Mansouri, 1998] and cells in the thymus [Peters, 1998], respectively. Pax-4/6 were expressed in the fore/midgut and gave rise to δ/β-cells [Sosa-Pineda, 1997] and α-cells [St-Onge, 1997], respectively. Pax-1 has been demonstrated to be involved in T-cell maturation [Wallin, 1996]. Mutation in Pax-1 resulted in reduced ability to mature CD4/8 negative thymocytes into CD4/8 positive thymocytes [Su, 2000]. Furthermore, pax-1 is expressed in the notochord to maintain proliferation of sclerotome cells during the development of the vertebral column [Furumoto, 1999]. In the present study Pax-1 was down-regulated (0.7) after maturation of the β-cell phenotype, suggesting that this transcription factor is no longer needed in the mature β-cell, but may have been involved earlier in the maturation of the β-cells.

Under physiological conditions, heat shock proteins (Hsp) are expressed in a constitutive manner, and exert housekeeping and homeostatic functions. They act as molecular chaperones playing a role in protein folding, transport, translocation and degradation (rewieved in [Langer, 1994]. Furthermore, Hsp may be activated after exposure to several toxic compounds such as heat [Gabai, 1993], cytokines [De Vera, 1996; Scarim, 1998] and free radicals (NO) [Bellmann, 1995; Bellmann, 1996], thus acting as a protective mechanism against these stress factors. Chaperoning functions of the Hsp-70 family members have been demonstrated to be dependent on the ATP level [Beckmann, 1990], since when the ATP level is depleted by an uncoupler (CCCP) Hsp 68/70 are induced [Gabai, 1993]. After β-cell maturation the proteins in the energy generation pathway are down-regulated (isocitrate 0.7, citrate synthase 0.2, voltage-dependent anion-selctive channel protein 1 0.3, cAMP-dependent protein kinase 0.4, isocitrate dehydrogenase 0.3, creatine kinase B 0.5 and G25 GTP-binding protein 0.5), which results in low ATP production. In contrast, to compensate for the low ATP level ATP synthase α chain was highly up-regulated (5.1). After β-cell maturation Hsp were up-regulated (HSc-70-ps1 (4.4/16.7), 78 Kda glucose-related protein (GRP78, 4.0/1.6/2.6), Hsc-70-interacting protein (2.9) and mortalin (GRP75, 16.7)) this could be a result of low ATP level. Despite the higher amount of Hsp in the β-cell phenotype compared to the pre-β-cell phenotype, it has been demonstrated in previous analysis that the β-cell phenotype is more sensitive to IL-1β [Nielsen, 1999]. In this context, Hsp has been demonstrated to be expressed 3-4 fold higher in human islets compared to rodents islets [Welsh, 1995], despite this, human islets are still sensitive to cytokines [Eizirik, 1996]. The higher amount of Hsp in the β-cells could reflect a defense mechanism activated by the β-cells to protect themselves against toxic compounds. In contrast, gluthatione-S-tranferase (GST) (0.5/0.3), which is involved in the glutathione pathway is down-regulated, suggesting that the β-cells are less able to reduce the toxic H₂O₂ compared to the pre-β-cell phenotype. Glutathione has been demonstrated to protect a human insulinoma cell-line against the toxic effect of tumor necrosis factor α (TNF-α) [Cavallo, 1997], and together with catalase, glutathione protects RIN cells against H₂O₂, reactive oxygen species and cytokines [Tiedge, 1998; Tiedge, 1999]. Another function of GST is inhibition of the Jun N-terminal kinase (JNK) activity [Adler, 1999]. JNK is activated in response of different stress factors such as cytokine, heat and oxidative compounds. Dependent upon the stimulation, signaling through JNK activates cell death (apoptosis), differentiation/proliferation or tumor development (reviewed in [Davis, 2000]. PKC-interacting cousin of thioredoxin (PICOT) is another JNK inhibitor [Witte, 2000], which was down-regulated (0.5) during β-cell maturation. After exposure to different stress factors the β-cells express a higher level of JNK activity due to low amount of both GST and PICOT, making the β-cells more sensitive to different stress factors compared to the preβ-cells.

Another specific function of Hsc70 is uncoating of clatrin-coated vesicles. Secreted proteins are transported in ciatrin-coated vesicles to the plasma membrane and before fusion of the vesicles with the plasma membrane the coat formed by clatrin triskelion is removed by Hsc70 [DeLuca-Flaherty, 1990]. The Hsc70 mediated uncoating of clatrin-coated vesicles is dependent upon ATP hydrolysis [Greene, 1190]. After maturation of the β-cell phenotype an increased level of clatrin light chain (5.2), a component in clatrin triskelion was detected suggesting an increased level of exocytosis in the β-cells. This correlates well with maturation of the β-cell phenotype and associated increased insulin-production secretion through exocytosis in clatrin-coated vesicles [Turner, 2000]. The ATP-synthase alpha chain was highly up-regulated (5.1/2.2 fold) during β-cell maturation. This may represent a mechanism to compensate for the low level of ATP and the need for ATP hydrolysis during exocytosis.

Mortalin, a member of the Hsp70 family, was highly up-regulated (16.7) during maturation of the β-cell phenotype. Mortalin was first identified as a 66 kDa protein present in cytosolic fragments of normal mouse and absent in immortal cells [Wadhwa, 1991]. Stable transfection with mortalin in NIH 3T3 cells induced senescence, suggesting an anti-proliferative role of this protein *in vitro* [Wadhwa, 1993]. Furthermore, mortalin has been demonstrated to associate with the IL-1 receptor type 1 in an ATP dependent process [Sacht, 1999]. As a consequence of this association it is suggested that activation of the IL-1 receptor type 1 cascade after IL-1β exposure is highly up-regulated, since the β-cell phenotype express a high amount of mortalin.

Programmed cell death (apoptosis) plays an important role during cell maturation (reviewed in [Ellis, 1991]). It has been characterized by a set of cellular events including cell shrinkage, chromatin condensation and DNA fragmentation. Many factors have been demonstrated to be involved in this process including FAS, bcl-2, lamins, ICE and other caspases (reviewed in [Schulze-Osthoff, 1998; Cohen, 1997]). Cytokines and other toxic compounds have been demonstrated to induce apoptosis [Kaneto, 1995; Friedlander, 1996; Delaney, 1997; Matteo, 1997; Karlsen, 2000]. After maturation of the β-cell phenotype both lamin A and B were up-regulated (1.4 and 2.6, respctively). The proteolysis of lamins, the major structural proteins of the nuclear envelope, is observed in different cells undergoing apoptosis [Oberhammer, 1994; Greidinger, 1996]. This suggests that the β-cells compared to the pre-β-cell phenotype may enter the apoptotic pathway more frequently dependent upon activation. It has been demonstrated that inhibitors of lamin cleavage prevent apoptosis [Lazebnik, 1995; Neamati, 1995].

TOAD 64 has been shown to be involved during neural development [Minturn, 1995], but its importance for β-cell maturation is still unknown. TOAD 64 has furthermore been characterized to make a complex of 5 proteins (NADH oxidoreductase homologues to GADPH, enolase c, enolase γ and Hsc70) [Bulliard, 1997] defied as the PMO complex, involved in cellular defense in response to oxidative stress. TOAD 64 was both up-regulated and down-regulated (2.6/1.6/0.6) after maturation of the β-cell phenotype, suggesting post-translational modification, which may result in several different functions.
Citrullinaemia is an autosomal disorder of the urea metabolism characterized by a high level of citrulline as a result of deficiency in the activity of the urea cycle enzyme argininosuccinate synthetase (ASS) [Kobayashi, 1991]. Ammonia (NH3) inters the urea cycle and is converted to citrulline, ASS catalyzes the reaction of citrulline to argininosuccinate, argininosuccinate is converted to arginine and the end product is urea [Rochvansky, 1967]. Defect or mutation in ASS causes high level of both NH₃ and citrulline. The expression level of ASS (0.2) was significantly lower in the β-cell phenotype compared to the prep-cell phenotype, resulting in an increased level of NH₃ and citrulline in the β-cell phenotype, which would make the β-cell phenotype more sensitive when exposed to cytokines and toxic compounds. Cytokines activate the formation of nitric oxide (NO), which may contribute to pancreatic β-cell damage. The inducible form of nitric oxide synthase (iNOS) catalyzes the conversion of arginine to citrulline and NO. Arginine can be provided extracellularly by protein degradation or synthesis from citrulline [Morris, 1994]. It is possible that due to the down-regulated ASS expression and resulting high level of citrulline in the β-cell phenotype, the β-cells may convert citrulline to arginine. The pool of arginine may then serve as substrate for iNOS and further production of NO and other free radicals derivates. Indeed it has been demonstrated that IL-1β exposed β-cells induce the citrulline-NO cycle, and extracellular arginine or citrulline are required for NO production [Flodström, 1999]. Furthermore, accumulation of arginine was shown to be higher in the IL-1β exposed β-cells compared to the control cells [Flodström, 1999]. It is possible that the higher concentration of citrulline in the β-cell phenotype due to low ASS is an acquired trait during β-cell maturation, which makes the β-cells more sensitive to IL-1β because the citrulline-NO cycle is increased.

A first aspect of the invention relates to a method for diagnosing diabetes in a human, the method comprising determining the presence or level of expression of at least one marker protein in a biological sample from the human, wherein the marker protein is selected from the group consisting of

**(Table 1)**

| **Gel spot no:** | **Protein** | **Database Acc #** | **MW** | **Theor MW** | **pl** | **Theor pl** |
|---|---|---|---|---|---|---|
| NEPHGE 76 | Phosphoglycerate kinase * | P16617 | 37.1 | 44.4 | 8.3 | 7.5 |
| NEPHGE 124, 193, 241, 105# | Fructose-bisphosphate aldolase A * | P05065 | 20.7, 35.4, 34.9, 35.6 | 39.2 | 8.6, 8.9, 8.9, 8.3 | 8.4 |
| NEPHGE 568 | Glyceraldehyde-3-phophate-dehydrogenase * # | P04797 | 35.7 | 35.7 | 7.8 | 8.4 |
| IEF 166* | Enolase α | P04764 | 49.6 | 47.0 | 5.7 | 6.2 |
| IEF 193, 1219 | Enolase γ* | P07323 | 47.8, 62.8 | 47.0 | 5.0, 5.1 | 5.0 |
| IEF 255* | Transaldolase | Q93092 | 37.7 | 37.4 | 6.1 | 6.6 |
| IEF 794 | Glyceraldehyde-3-phophate-dehydrogenase | M17701 | 35.9 | 35.7 | 6.8 | 8.4 |
| IEF 1472*, 1473* | Puruvate kinase, M1 isozyme | P11980 | 53.5, 53.5 | 57.7 | 7.2, 7.1 | 6.7 |
| NEPHGE 77* | Argininosuccinate synthase | P00966 | 40.1 | 46.4 | 8.1 | 8.4 |
| NEPHGE 105# | Heterogeneous nuclear ribonucleoprotein A3 | P51991 | 35.6 | 39.7 | 8.3 | 8.7 |
| NEPHGE 105# | Poly (RC) binding protein 2 | Q61990 | 35.6 | 38.2 | 8.3 | 6.3 |
| NEPHGE 230* | EIF-2-gamma Y | Q9Z0N2 | 42.6 | 51.0 | 8.8 | 8.8 |
| NEPHGE 357# | 40S ribisomal protein S25 | P25111 | 20.1 | 13.7 | 7.7 | 10.1 |
| NEPHGE 357# | 40S ribosomal protein S18 | P25232 | 20.1 | 17.7 | 7.7 | 11.0 |
| NEPHGE 551 | Heterogeneous nuclear ribonucleoprotein A2/B1 # | P22626 | 35.1 | 37.4 | 9.0 | 9.0 |
| NEPHGE 4410* | 60S ribisomal protein L26 | P12746 | 21.0 | 17.3 | 8.0 | 10.6 |
| NEPHGE 4410* | Ubiquitin-conjucating enzyme E2 | 076069 | 21.0 | 20.9 | 8.0 | 7.6 |
| IEF 255* | 60S Acidic ribisomal protein P0 | P19945 | 37.7 | 34.2 | 6.1 | 5.9 |
| IEF 256 | Pyridoxal kinase | 035331 | 39.1 | 34.9 | 6.3 | 6.3 |
| IEF 383* | Isovaleryl-CoA dehydrogenase | P12007 | 46.4 | 46.4 | 6.2 | 8.0 |
| IEF 383* | Ubiquitin fusion degradetion protein 1 homolog | P70362 | 46.4 | 34.5 | 6.2 | 7.0 |
| IEF 403, 1039*, 1500* | 26S protease regulatory subunit 7 | Q63347 | 48.6, 36.8, 39.5 | 48.6 | 5.6, 5.8, 5.8 | 5.6 |
| IEF 12315* | Translation initiation factor 5 | Q07205 | 77.7 | 49.0 | 4.8 | 5.4 |
| NEPHGE 14 | Isocitrate dehydrogenase | P54071 | 39.8 | 58.7 | 8.6 | 8.9 |
| NEPHGE 77* | Citrate synthase | 075390 | 40.1 | 51.7 | 8.1 | 8.1 |
| NEPHGE 252, 4234* | Voltage-dependent anion-selective channel protein 1 | Q60932 | 30.6, 30.5 | 30.6 | 8.0, 8.0 | 8.6 |
| NEPHGE 335* | Phosphoenolpyruvate carboxylase | P29195 | 22.4 | 109.4 | 7.6 | 5.8 |
| NEPHGE 377, 516 | ATP synthase alpha chain # | P15999 | 56.8, 51.6 | 58.8 | 8.0, 8.1 | 9.2 |
| NEPHGE 582, 4234*, 45124 | Voltage-dependent anion-selective channel protein 2 # | P81155 | 31.6, 30.5, 32.6 | 31.7 | 7.4, 8.0, 7.9 | 7.4 |
| NEPHGE 20140 | Fumarate hydratase | P14408 | 42.9 | 54.5 | 8.1 | 9.1 |
| IEF 123 | cAMP-depend. protein kinase type I-alpha regu. chain | P09456 | 48.3 | 43.0 | 5.3 | 5.3 |
| IEF 359 | Isocitrate dehydrogenase | P41562 | 48.7 | 46.7 | 6.5 | 6.5 |
| IEF 616* | Creatine kinase, B chain # | P07335 | 43.5 | 42.7 | 5.3 | 5.3 |
| IEF 700, 1296 | G25 GTP-binding protein | P25763 | 21.3, 23.4 | 21.3 | 6.1, 6.2 | 6.2 |
| NEPHGE 59* | RAN | P17080 | 24.9 | 37.8 | 8.0 | 9.4 |
| NEPHGE 156*, 303* | T-complex protein 1, beta subunit | Q99832 | 47.5, 47.7 | 59.4 | 8.4, 8.3 | 7.6 |
| NEPHGE 332 | RAS-related protein RAB-5C | P51148 | 23.6 | 23.6 | 8.3 | 8.9 |
| NEPHGE 453 | Peptidyl-prolyl cis-trans isomerase A | P10111 | 17.7 | 17.7 | 8.4 | 8.4 |
| IEF 82, 85*, 1463* | Hsc 70-ps1 | CAA49670 | 61.9, 72.3, 62.0 | 70.9 | 5.4, 5.1, 5.4 | 5.4 |
| IEF 85*, 775*, 846*, 1358 | 78 Kda glucose-related protein * # | P06761 | 72.3, 70.0, 40.5, 96.0 | 72.3 | 5.1, 5.1, 6.1,4.9 | 5.1 |
| IEF 109*, 542, 806, 973* | Probable protein disulfide isomerase ER-60 * # | P11598 | 54.7, 59.5, 24.1, 58.3 | 56.6 | 5.6,5.7, 4.6, 6.3 | 5.9 |
| IEF 109* | T-complex protein 1, theta sububit | P42932 | 54.7 | 59.6 | 5.6 | 5.4 |
| IEF151 | ERJ3 protein | Q9UBS4 | 49.7 | 40.5 | 6.1 | 5.8 |
| IEF 376 | N-ethylmaleimide sensitive factor | Q9QUL6 | 65.2 | 82.7 | 6.4 | 6.6 |
| IEF 408 | Clatrin light chain | AAA40891 | 59.6 | 25.1 | 4.6 | 4.6 |
| IEF 463 | RAS-related protein RAB-11B | P46638 | 25.2 | 24.5 | 6.3 | 5.6 |
| IEF 469#, 1472*, 1473* | T-complex protein, zeta subunit* | P80317 | 59.7, 53.5, 53.5 | 58.0 | 6.3,7.2, 7.1 | 6.6 |
| IEF 583 | Vesicular-fusion protein NSF | P46460 | 64.5 | 82.6 | 6.4 | 6.5 |
| IEF 728^, 881# | T-complex protein 1, gamma subunit | P49368 | 67.4, 62.9 | 60.3 | 6.0, 6.2 | 6.2 |
| IEF 728^, 469#, 881#, NEP 282 | P60 protein | 035814 | 67.4, 59.7, 62.9,61.1 | 62.6 | 6.0,6.3, 6.2, 7.4 | 6.4 |
| IEF 730 | Hsc 70-interacting protein | P50503 | 49.5 | 41.3 | 5.1 | 5.3 |
| IEF 871*, 728^ | Coatomer delta subunit (bovin, human)* | P53619 | 70.1,67.4 | 57.2 | 6.0, 6.0 | 5.9 |
| IEF 922 | Kinesin heavy chain | P33176 | 92.1 | 109.9 | 5.9 | 6.1 |
| IEF 1014* | Amphiphysin-like protein | 008839 | 84.2 | 64.5 | 5.0 | 5.0 |
| IEF 1039*, 1500* | Sorting Nexin 6 | Q9UNH7 | 36.8, 39.5 | 46.6 | 5.8, 5.8 | 5.8 |
| IEF 1451* | Apolipoprotein A-I | P02647 | 58.2 | 30.8 | 6.9 | 5.6 |
| IEF 1463* | Mortalin (GRP75)* | P48721 | 62.0 | 73.9 | 5.4 | 6.0 |
| IEF 1513 | Alpha-soluble NSF attachment protein | P54921 | 16.0 | 33.2 | 6.0 | 5.3 |
| IEF 9224 | Heat-shock protein 105 Kda | Q61699 | 87.6 | 96.5 | 5.5 | 5.4 |
| NEPHGE 356 | Transgelin 2 | P37802 | 22.4 | 22.4 | 8.2 | 8.4 |
| NEPHGE 447 | Neurofilament triplet H protein | P16884 | 21.3 | 89.5 | 7.9 | 5.6 |
| NEPHGE 454 | Complement component C4 | Q29439 | 18.0 | 14.5 | 8.3 | 5.3 |
| NEPHGE 454 | Destrin | JE0223 | 18.0 | 18,4 | 8.3 | 7.8 |
| NEPHGE 19991 | Caldesmon | Q05682 | 58.8 | 93.3 | 8.1 | 5.6 |
| IEF 104, 612, 616* | Keratin, type II cytoskeletal 8 # | Q10758 | 53.9, 45.3, 43.5 | 53.9 | 5.7, 5.5, 5.3 | 5.8 |
| IEF 202, 1193 | Alpha-2-macroglobulin receptor-associated protein | Q99068 | 43.5, 45.4 | 41.7 | 6.5, 6.7 | 6.9 |
| IEF 215 | Serine/threonine protein phosphatase PP1-beta | P37140 | 37.2 | 37.2 | 6.2 | 5.8 |
| IEF 232 | PKCq-interacting protein PICOT | AAF28843 | 40.9 | 31.4 | 5.8 | 4.9 |
| IEF 330* | Cofilin, non-muscle isoform | P45592 | 18.3 | 18.5 | 6.5 | 8.2 |
| IEF 469# | Dihydropyrimidase related protein-1 (CRMP-1) | Q62950 | 59.7 | 62.2 | 6.3 | 6.6 |
| IEF 565, 12315*, 12340 | Protein disulfide isomerase | P04785 | 86.6, 77.7, 116.3 | 57.0 | 4.9, 4.8, 5.0 | 4.8 |
| IEF 604, 1438 | Ezrin | P26040 | 76.2, 81.0 | 69.2 | 6.0, 5.8 | 5.8 |
| IEF 662 | Nonmuscle myosin heavy chain-B | AAF61445 | 94.4 | 22.9 | 5.7 | 5.5 |
| IEF 900 | Reticulocalbin 1 | Q05186 | 105.7 | 38.1 | 4.7 | 4.7 |
| IEF 728^, 871*, 881# | Turned on after division 64 (TOAD 64) (CRMP-3) * # | P47942 | 67.4, 70.1, 62.9 | 62.3 | 6.0, 6.0, 6.2 | 6.0 |
| IEF 935, 1014* | Endoplasmin | P08113 | 98.5, 84.5 | 92.5 | 4.7, 5.0 | 4.7 |
| IEF 973*, 1351 | Lamin A* | P48679 | 58.3, 66.0 | 74.3 | 6.3, 6.2 | 6.5 |
| IEF 1020 | Myosin heavy chain | Q90337 | 72.4 | 221.1 | 4.6 | 5.6 |
| IEF 1154 | Lamin B1 | P70615 | 68.4 | 66.6 | 5.2 | 5.2 |
| IEF 1451* | Fibrinogen gamma-a chain | P02679 | 58.2 | 49.5 | 6.9 | 5.6 |
| IEF 1482 | Vitamin D-binding | P04276 | 46.6 | 53.5 | 5.5 | 5.7 |
| IEF 1564 | Fatty acid-binding protein, epidermal | P55053 | 11.6 | 15.1 | 6.3 | 6.7 |
| NEPHGE 59* | PAX 1 | P15863 | 24.9 | 24.4 | 8.0 | 6.6 |
| NEPHGE 156* | Lamina-associated poypeptide 2 | Q62733 | 47.5 | 50.3 | 8.4 | 9.4 |
| NEPHGE 230*, 20127# | Flag structure-specific endonuclease | AAF81265 | 42.6, 41.2 | 42.6 | 8.8, 8.6 | 8.8 |
| NEPHGE 357# | RNA polymerase II transscriptional coactivator P15 | Q63396 | 20.1 | 13.7 | 7.7 | 9.7 |
| NEPHGE 458 | Lupus la protein homolog | P38656 | 19.4 | 47.8 | 8.0 | 9.7 |
| NEPHGE 526 | DNA-polymerase | P78988 | 42.9 | 99.5 | 9.4 | 8.9 |
| NEPHGE 19980, 45036 | Septin-like protein | Q9QZR6 | 54.5, 64.4 | 63.8 | 8.4, 7.8 | 8.7 |
| NEPHGE 20127# | Hypothetical 44.7 protein | CAB66481 | 41.2 | 44.7 | 8.6 | 7.6 |
| NEPHGE 20127# | CDC10 protein homolog | Q9WVC0 | 41.2 | 50.5 | 8.6 | 8.8 |
| IEF 156 | NEDD 5 protein | P42208 | 38.0 | 41.5 | 6.1 | 6.1 |
| IEF 166* | Histidyl-tRNA synthetase | Q61035 | 49.6 | 57.4 | 5.7 | 5.7 |
| IEF 313 | Zinc Finger protein 43 | P28160 | 27.2 | 93.5 | 6.4 | 9.4 |
| IEF 330* | Nucleoside diphophate kinase B | P19804 | 18.3 | 17.3 | 6.5 | 6.9 |
| IEF 462 | Cytidylate kinase # | P30085 | 23.3 | 22.2 | 6.3 | 5.4 |
| IEF 775* | Heterogeneous nuclear ribonucleoprotein K | Q07244 | 70.0 | 51.0 | 5.1 | 5.4 |
| IEF 846* | Zinc finger protein 26 | P10076 | 40.5 | 48.9 | 6.0 | 9.3 |
| IEF 850* | Reverse transcriptase | Q9YQW2 | 57.8 | 27.9 | 6.1 | 9.3 |
| IEF 885 | Importin alpha | Q9Z0N9 | 48.5 | 57.8 | 5.4 | 5.4 |
| IEF 1209 | FUSE binding protein 2 | Q92945 | 71.7 | 68.4 | 6.5 | 8.5 |
| IEF 5223 | Dynactin, 50 Kda isoform | Q13561 | 50.2 | 44.8 | 5.1 | 5.1 |
| NEPHGE 303* | Coding region determinant binding protein | 088477 | 47.7 | 63.5 | 8.3 | 9.3 |
| NEPHGE 335* | Polyubiquitin | Q63654 | 22.4 | 11.2 | 7.6 | 5.4 |
| NEPHGE 441 | Glutathione S-transferase P | P46524 | 23.5 | 23.5 | 8.0 | 8.1 |
| NEPHGE 605 | Glutathione S-transferase YB1 | P04905 | 25.9 | 25.9 | 8.8 | 8.4 |
| IEF 482 | Neurolysin | P42676 | 80.3 | 80.3 | 5.6 | 6.0 |
| IEF 850* | Proteasome component C2 | P18420 | 57.8 | 29.5 | 6.1 | 6.1 |
| IEF 1508 | Arginase 1 | P07824 | 43.1 | 35.0 | 6.8 | 6.8 |

and marker proteins further consisting of modifications and derivatives of marker proteins of Table 1, so as to have at least 80% homology with marker proteins of Table
1,
wherein pI is the isoelectric point of the marker protein as determined by isoelectric focusing, and the molecular weight (MW) is determined on a polyacrylamide gel.

A further aspect relates to a method for diagnosing diabetes in a human, wherein the method further comprises establishing the increased expression of at least one marker protein (an up-regulated marker protein) or establishing the decreased expression of at least one marker protein (a down-regulated marker protein) selected from the group consisting of proteins or combinations of up- and down-regulated marker proteins.

The invention further relates to a method of treating diabetes by the up-regulation of a down-regulated protein, the down-regulation of an up-regulated protein, or combinations thereof. That is to say that the invention relates to a method of treating diabetes in a human comprising altering the expressing of marker proteins of Table 1. Furthermore, the invention relates to method of treating diabetes in a human comprising administering a marker protein of Table 1, a nucleotide sequence coding for a marker protein of Table 1, an antibody for a protein of Table 1, a nucleic acid fragment capable of binding to a marker protein of Table 1, or a compound capable of binding to a marker protein of Table 1 to said human.

A further aspect relates to the use of novel proteins and proteins of Table 1 as markers or indicators for diabetes as well as to the use of known proteins whose presence, absence or prevalence has previously not been associated with diabetes. The changes in protein expression and patterns of protein expression are considered to be important markers for diagnosis, prognosis and therapeutic applications and targets.

The method of the present invention may be further used to determine the predisposition in a human for diabetes, the method comprising determining the presence or relative level in a biological sample from the human of at least one marker protein wherein the marker protein being indicative of a predisposition for having diabetes is selected from the group consisting of (Table 1) and marker proteins further consisting of modifications and derivatives of marker proteins of Table 1, so as to have at least 80% homology with marker proteins of Table 1, wherein pI is the isoelectric point of the marker protein as determined by isoelectric focusing, and the molecular weight (MW) is determined on a polyacrylamide gel.

A method for diagnosing the predisposition in a human for diabetes, may comprise determining the increased expression in a biological sample from the human of at least one marker protein selected from the a biological sample from the human, said marker protein selected from the group consisting of proteins of Table 2 establishing the decreased expression of at least one marker protein (a down-regulated marker protein) in a biological sample from the human, or combinations of up- and down-regulated marker proteins.

Thus, the determination of whether a protein is up-regulated or down-regulated serves as useful indicators of diabetes susceptibility. The pattern of up and down regulation may also serve as an indicator. That is to say that the level of expression of more than one protein is established and the pattern of expression of a grouping of proteins is used as an indicator.

In a suitably embodiment, at least one marker protein is selected from the group consisting of one or more proteins present in a significantly lower or significantly higher amount on a polyacrylamide gel of proteins from said biological sample in relation to a control, one or more proteins present on a polyacrylamide gel of proteins from said biological sample and absent on polyacrylamide gel of proteins of a control, one or more proteins absent on a polyacrylamide gel of proteins from said biological sample and present on polyacrylamide gel of proteins of a control.

Similarly, with regards to a method of treating diabetes, a single protein may be targeted for therapy or a grouping of proteins may be targeted. The level of expression of these targeted proteins may be altered or the proteins themselves may be interfered with in order to alter their activity. Thus, an interesting embodiment of a method of treating diabetes in a human comprises altering the expressing of a marker protein of Table 1. 9. A method of treating diabetes in a human comprising administering a marker protein of Table 1, a nucleotide sequence coding for a marker protein of Table 1, an antibody for a protein of Table 1, a nucleic acid fragment capable of binding to a marker protein of Table 1, or a compound capable of binding to a marker protein of Table 1 to said human.

A method of preventing or delaying the onset or of diabetes in a human according to the present invention may comprise administering a marker protein of Table 1, a nucleotide sequence coding for a marker protein of Table 1, an antibody for a protein of Table 1, a nucleic acid fragment capable of binding to a marker protein of Table 1, or a compound capable of binding to a marker protein of Table 1 to said human.

Thus a particularly interesting aspect of the present invention relates to a pharmaceutical composition which comprises a substance which is capable of regulating the expression of a nucleic acid fragment coding for at least part of a protein of Table 1, or at least one marker protein in Table 1, an antibody for a protein of Table 1, a nucleic acid fragment capable of binding to a marker protein of Table 1, or a compound capable of binding to a marker protein of Table to said human.

The invention further relates to a method of determining the likelihood of an agent having a therapeutic effect in the treatment of diabetes comprising determining the level of expression of one or more proteins of Table 1 before and after exposing a test model to said agent and comparing said levels.

In the testing of compounds, knowledge about the activity or target of an agent is useful for understanding the therapeutic activity of said agent and may assist in improving the desired therapy. The developments of the present investigators allows for a method of determining the effect of a compound in the treatment of diabetes comprising determining the level of expression of proteins of one or more proteins of Table 1 and to a method of determining the level of effect or level of activity of a compound used in the treatment of diabetes comprising determining the level of expression of one or more proteins of Table 1 before and after exposing a test model to said agent.

Thus, the invention further relates to a method for determining the physiological effect of a substance, the method comprising using a mammal which has been established to be an individual having a high likelihood of having diabetes or a genetic predisposition for having diabetes by use of the method according to the invention, the method comprising administering the substance to the individual and determining the effect of the substance.
The present investigators anticipate that a method of determining the nature or cause of diabetes in a human having or susceptible to said disease comprising establishing the level of expression of a protein of Table 1 in relation to a model serves for understanding the disease and potential therapies.

A further interesting application of the present invention would be the construction of transfected cells with one or more of the genes identified here with the goal to produce a cell with desirable characteristics that could mimic the normal function of the β-cell in the health condition. These cells could then be used for transplantation or introduction into the human organism for cell therapy (as for bone transplants). The cells used could be modified so that the immune system does not recognise them as foreign (possibly the patients own cells).
Furthermore the cells could be of β-cell or non β-cell origin (e.g. α-cell, stem cell, pleuripotent cell). Suitable regulatory elements would need to be inserted together with the genes - and one source of these might be the natural regulatory element for the genes themselves. This would result in a long lasting therapy for the patient.

Each of the methods of the present invention relates to the use of a protein according to Table 1 or having least 80% homology therewith.

The invention further relates to a nucleic acid fragment comprising a nucleotide sequence which codes for a peptide defined in Table 1 as well as to a nucleic acid fragment which hybridises with said nucleic acid fragment or a part thereof. The use of said nucleic acid fragment may serve to detecting the presence of a peptide of Table 1.

The invention further relates to an antibody able to bind to a protein defined in Table 1.
The antibody may be a polyclonal antibody or a monoclonal antibody. The use of an antibody may serve for detecting the presence of a peptide shown in Table 1.

An interesting aspect of the present invention relates to a test kit for diagnosing diabetes or a genetic predisposition for diabetes in a mammal, comprising:
a) a binding mean which specifically binds to at least one marker protein shown in Table 1 or an antibody for a protein of Table 1, a nucleic acid fragment capable of binding to a marker protein of Table 1, or a compound capable of binding to a marker protein of Table 1 to said human,
b) means for detecting binding, if any, or the level of binding, of the binding means to at least one of the marker proteins or at least one of the peptides or at least one of the nucleic acid fragments, and
c) means for correlating whether binding, if any, or the level of binding, to said binding means is indicative of the individual mammal having a significantly higher likelihood of having diabetes or a genetic predisposition for having diabetes.

### EXAMPLES

### Example 1

### Cell culture

The NHI-cell system [Nielsen, 1999] is based on the subclone NHI-glu derived from the glucagon producing MSL-G2 culture [Madsen, 1986]. Following *in vivo* passage by transplantation in syngeneic NEDH rats, the NHI-glu maturates into insulinomas [Madsen, 1988; Madsen, 1993; Blume, 1995]. Insulinomas re-established *in vitro* display a more mature insulin producing phenotype (NHI-ins) for prolonged periods [Serup, 1995], which closely resembles β**-**cells with respect to the mRNA expression profile [Jensen, 1996].

The two NHI-phenotypes were cultured in RPMI 1640 Glutamax (GibcoBRL) supplemented with 10 % FCS (GibcoBRL) and 1 % penicillin/streptomycin (GibcoBRL) at 37°C in a 5 % CO₂ atmosphere. For 2D-gel electrophoresis 2x10⁵ cells/well were cultured in 24 well plates (Costar, Cambridge, USA) and for protein identification by mass spectrometry (MALDI) 1x10⁵ cells/well were cultured in 96 well plates and 20x10⁶ cells/bottle were cultured in tissue culture flasks with and without [³⁵S]-methionine, respectively.

### Cell labeling

The cells were cultured in 68 h to allow cells to grow in 24 well plates. Then the cells were washed twice in HBSS and labeled for 4 h in 250 µl/well methionine-free Dulbecco's modified Eagle's medium (DMEM) [Andersen, 1995] with 10 % NHS dialyzed for amino acid, and 500 µCi/ml [³⁵S]-methionine (Amersham Corp.). To eliminate 2-mercaptoethanol, [³⁵S]-methionine was freeze-dried 24 h before labeling. After labeling the cells were washed twice in HBSS, pelleted, lysed with 100 µl lysis buffer (8.5 M urea, 2% nonidet P-40, 5% 2-mercaptoethanol and 2% carrier ampholytes, pH range 7-9) and frozen at -80°C.

### Determination of [³⁵S]-methionine incorporation

The amount of [³⁵S]-methionine incorporation was quantitated in duplicate by adding 10 µL BSA (0.2 µg/mL H₂O) as a carrier to 5 µL of a 1:10 dilution of each sample, followed by 0.5 mL of 10% TCA. This was left to precipitate for 30 min at 4°C before being filtered through 0.25 µm filters. The HAWP filters were dried and placed into scintillation liquid for counting.

### 2D-gel electrophoresis

The procedure was essentially as previously described [O'Farrell, 1977; Fey, 1984; Fey, 1997]. Briefly, first-dimensional gels contained 4% acrylamide, 0.25% bisacrylamide and carrier ampholytes (the actual ratio depending upon the batch) and were 175 mm long and 1.55 mm in diameter. An equal number of counts (10⁶ cpm) of each sample were applied to the gels. In case of lower amounts of radioactivity it was necessary to regulate the exposure time of the gel so that comparable total optical densities were obtained. The samples were analyzed on both isoelectric focusing (IEF; pH 3.5-7) and nonequilibrium pH-gradient electrophoresis (NEPHGE; pH 6.5-10.5) gels. IEF gels were prefocused for approximately 4 h at 140 µA/gel (limiting current); the sample was then applied and focused for 18 h at 1200 V (limiting voltage). NEPHGE gels were focused for approximately 6.5 h using 140 µA/gel and 1200 V as the limiting parameters. Second-dimension gels, 1 x 200 x 185 mm, contained either 15% acrylamide and 0.075% Bis, or 10% acrylamide and 0.05% Bis, and were run overnight. This separation protocol was optimized for hydrophilic proteins, thus a detailed characterization of hydrophobic (membrane) proteins is not possible. After electrophoresis, the gels were fixed in 45% methanol and 7.5% acetic acid for 45 min and treated for fluorography with Amplify® for 45 min before being dried. The gels were placed in contact with X-ray films and exposed at -70° C for 1-40 days. Each gel was exposed for at least three time periods to compensate for the lack of dynamic range of X-ray films.

### 2D-gel analyzing and statistical analysis:

The Bio Image computer program (version 6.1) was used to identify and quantitate protein-spots. The computer program assist in the matching of the spots between the four independent gels in a composite image, but further manual editing is necessary to ensure correct matching of computer found spots. After correct matching of the entire computer found spots statistical analysis were used to analyze the significantly changed % IOD level after maturation from the NHI-glu to the NHI-ins phenotype. For statistical evaluation a double-sided non-paired t-test was used and the level of significance was chosen at p<0.01.

### Protein characterization

Preparatory 2D-gels were produced from the pool of cells, prepared and separated on gels as described above. For localization of the spots, 10% of the cells were radioactively labeled and used as tracer. Since initial attempts to identify the proteins in the gel resulted in very few positive identifications by direct micro sequencing, the method of choice became mass spectrometry.

### Protein identification by mass spectrometry (MALDI)

Briefly, protein spots of interest were obtained by cutting them out of the dried gel using a scalpel. One hundred and thirty five spots could technically be cut out of the gels for analysis. The proteins were enzymatically digested in the gel as described [Rosenfeld, 1992; Shevchenko, 1996] with minor modifications [Nawrocki, 1998]. The excised gel plugs were washed in 50 mM NH₄HCP₃/acetonitrile (60/40) and dried by vacuum centrifugation. Modified porcine trypsin (12 ng/µL, Promega, sequencing grade) in digestion buffer (50 mM NH₄HCO₃) was added to the dry gel pieces and incubated on ice for 1 h for reswelling. After removing the supernatant, 20-40 µL digestion buffer was added and the digestion was continued at 37° C for 4-18 hours. The peptides were extracted as described [Shevchenko, 1996] and dried in a vacuum centrifuge. The residue was dissolved in 5 % Formic acid and analyzed by matrix assisted laser desorption/ionization (MALDI) mass spectrometry. Delayed extraction MALDI mass spectra of the peptide mixtures resulting from in-gel digestion were acquired using a PerSeptive Biosystems Voyager Elite reflector time-of-flight mass spectrometer (Perseptive Biosystems, Framingham, MA). Samples were prepared using α-cyano-4-hydroxy cinnamic acid as matrix. When appropriate, nitrocellulose was mixed with the matrix [Kussmann, 1997]. Protein identification was performed to search for the peptide-mass maps in a comprehensive, non-redundant protein sequence database (NRDB, European Bioinformatics Institute, Hinxton, UK) using the PeptideSearch software ([Mann, 1993] further developed at EMBL (Heidelberg, Germany)). The protein identifications were examined using the "second pass search" feature of the software and critical evaluation of the peptide mass map as described [Jensen, 1998]. The following protein databases were searched for matches: SWISS-PROT, PIR, NIH, and GENEBANK

### Determination of Mw and pI

Theoretical pI and Mw were calculated using the 'Compute pI/Mw tool' at the ExPASy Molecular Biology Server (www.expasy.ch/tools/pi tool.html). pI/Mw for the individual proteins on the gel were determined by plotting the theoretical pI/Mw against the running length of the gel. The proteins outside the line were removed and proteins on the line were used in the Bioimage Program to calculate all the unknown pI and Mw on the gel.

### LEGEND TO TABLE 1

In Table 1 the spot numbers refer to the numbers assigned by the Bio Image computer program, when the gels are matched together. Protein-spots containing 2, 3 or 4 proteins are demonstrated by *, # and ^, respectively (column 1). The specific protein matching the database Acc. number is assigned according to the major known function of the specific protein. Proteins assigned by * has been shown also to be altered after IL-1β exposure of Wistar Wurth rat islets, and proteins assigned by # were also altered after IL-1β exposure of BB rat islets (column 2). The IOD ratio of the protein is given relative to the expression level in the pre-β-cell phenotype, thus values below 1 represent proteins that are down-regulated and values above 1 represent proteins that are up-regulated during maturation from the pre-β-cell to the β-cell phenotype. Theoretical pI and Mw were calculated using the 'Compute pI/Mw tool' at the ExPASy Molecular Biology Server and the observed pI and Mw are defined in the methods.

### REFERENCES

1. Gepts W: Pathologic anatomy of the pancreas in juvenile diabetes mellitus. Diabetes 14:619-633,1965.
2. Junker K, Egeberg J, Kromann H, Nerup J: An Autopsy Study of the Islets of Langerhans in Acute Onset Juvenile Diabetes Mellitus. Acta Pathologica Et Microbiologica scandinavica Section a Pathology 85:699-706,1977
3. Nerup J, Mandrup-Poulsen T, Helqvist S, Andersen HU, Pociot F, Reimers JI, Cuartero BG, Karlsen AE, Bjerre U, Lorenzen T: On the pathogenesis of IDDM. Diabetologia 37 (suppl 2):S82-89,1994
4. Corbett JA, McDaniel ML: Intraislet release of intedeukin 1 inhibits p cell expression of inducible nitric oxide synthase. J Exp Med 181:559-568,1995
5. Mandrup-Poulsen T: The role of interleukin-1 in the pathogenesis of insulin-dependent diabetes mellitus. Diabetologia 39:1005-1029,1996
6. Lortz S, Tiedge M, Nachtwey T, Karlsen A, Nerup J, Lenzen S: Protection of insulin-producing RINm5F cells against cytokine-mediated toxicity through overexpression of antioxidant enzymes. Diabetes 49:1123-1130, 2000
7. Mose Larsen P, Fey SJ, Larsen MR, Nawrocki A, Andersen HU, Kähler H, Heilmann MCV, Roepstorff P, Pociot F, Kadsen AE, Nerup J: Proteome analysis of IL-β induced changes in protein expression in rat islets of Langerhans. Diabetes, In press 2001
8. Bone AJ: Animal models of type I diabetes, Current Opinion in Oncologic, Endocrine and Metabolic Investigational Drugs 2:192-200, 2000
9. Nakhooda AF, Like AA, Chappel Cl, Murray FT, Marliss EB: The spontaneously diabetic Wistar rat. Metabolic and morphologic studies. Diabetes 26:100-112,1977
10. Andersen HU, Mandrup-Poulsen T, Egeberg J, Helqvist S, Nerup J: Genetically determined differences in newborn rat islet sensitivity to interleukin-1 in vitro: no association with the diabetes prone phenotype in the BB-rat. Acta endocrinologica 120:92-98,1989
11. Reimers JT, Andersen HU, Mauricio D, Pociot F, A.E. K, Petersen JS, Mandrup-Poulsen T, Nerup J: Strain dependent differences in sensitivity of rat beta-cells to IL-1 beta in vitro and in vivo: Association with islet nitric oxide synthesis. Diabetes 45:771-778,1996
12. Bellmann K, Hui L, Radons J, Burkart V, Kolb H: Low stress response enhances vulnerability of islet cells in diabetes-prone BB rats. Diabetes 46:232-236,1997
13, Christensen UB, Larsen PM, Fey SJ, Andersen HU, Nawrocki A, Sparre-T., Mandrup-Poulsen T, Nerup J: Islet protein expression changes during diabetes development in islet syngrafts in BB-DP rats and during rejection of BB-DP islet allografts. Autoimmunity 32:1-15, 2000
14. Andersen HU, Fey SJ, Mose Larsen P, Nawrocki A, Hejnæs KR, Mandrup-Poulsen T, Nerup J: Interleukin-1 beta induced changes in the protein expression of rat islets. Electrophoresis 18:2091-2103, 1997
15. Brunstedt J, Nielsen JH, Lemmark A, and The Hagedom Study Group: Isolation of islets from mice and rats, in Methods in diabetes research, (Laboratory methods, part C) (vol 1), edited by Larner J, Pohl SL, New York, Wiley & Sons, 1984, pp 254-288
16. O'Farrell PZ, Goodman HM, O'Farrell PH: High resolution two dimensional electrophoresis of basic as well as acidic proteins. Cell 12:1133-1142,1977
17. Fey SJ, Nawrocki A, Larsen MR, Gorg A, Roepstorff P, Skews GN, Williams R, Mose Larsen P: Proteome analysis of Saccharomyces cerevisiae: a methodological outline. Electrophoresis 18:1361-1372,1997
18. Fey SJ, Mose Larsen P, Biskjær N: The protein variation in basal cells and certain basal cell related benign and malignant diseases, Faculty of Natural Science, University of Arhus, Denmark, 1984
19. Rosenfeld J, Capdevielle J, Guiliemot JC, Ferrara P: In-gel digestion of proteins for internal sequence analysis after one- or two-dimensional gel electrophoresis. Anal. Biochem 203:173-179, 1992
20. Shovchenko A, Wilm M, Vorm 0, Mann M: Mass spectrometric sequencing of proteins from silver stained polyacrylamide gels. Anal. Chem 68:850-858,1996
21. Nawrocki A, Larsen MR, Podtelejnikov AV, Jensen ON, Mann M, Roepstorff P, Gorg A, Fey SJ, MoseLarsen P: Correlation of acidic and basic, ampholyte and immobilised pH gradient 2D gel patterns based on mass spectrometric identification. Electrophoresis 19:1024-1035,1998
22. Kussmann M, Nordhoff E, Nielsen HR, Haebel S, Larsen MR, Jacobsen L, Jensen C, Gobom J, Mirgorodskaya E, Kristensen AK, Palm L, Roepstorff P: MALDI-MS sample preparation techniques designed for various peptide and protein analytes. Joumal of Mass Spectrometry 32:593601,1997
23. Mann M, Højrup P, Roepstorff P: Use of Mass Spectrometric Molecular Weight Information to Identify Proteins in Sequence Databases. Biol. Mass Spectrom 22:338-345,1993
24. Jensen ON, Larsen MR, Roepstorff P: Mass spectrometric identification and microcharacterization of proteins from electrophoretic gels: Strategies and applications. Proteins: Structure, Function and Genetics 33:74-89,1998
25. Asayama K, Kooy NW, Burr IM: Effect of vitamin R deficiency and selenium deficiency on insulin secretory reserve and free radical scavenging systems in islets: decrease of islet manganosuperoxide dismutase. Journal of laboratory and clinical medicine 107:459-464,1986
26. Sumoski W, Paquerizo H, Rahinovitch A: Oxygen Free Racal Scavengers Protect Rat Islet Cells from Damage by Cytokines. Diabetologia 32:792-796,1989
27. Welsh N, Bendtzen K, Sandler S: Influence of protease on inhibitory and stimulatory effects of interleukin 1 beta on beta-cell function. Diabetes 40:290-294,1991
28, Andersen HU, Mose Larsen P, Fey SJ, Karisen AE, Mandrup-Poulsen T, Nerup J: Two-dimensional gel electrophoresis of rat islet proteins. Interleukin 1 beta-induced changes in protein expression are reduced by L-arginine depletion and nicotinamide. Diabetes 44:400-407,1995
29. Helqvist S, Polla SS, Johannesen J, Nerup J: Heat shock protein induction in rat pancreatic islets by recombinant human interleukin 1 beta. Diabetologia 34:150-156,1991
30. Borg LAH, Cagliero E, Sandler S, Welsh N, Eizirik DL: Interleukin-1β increases the activity of superoxide dismutase in rat pancreatic islets. Endocrinology 130:2851-2857, 1992
31. Corbett JA, Lancaster JR, Sweetland MA, McDaniel ML: Intedeukin-1 beta-induced formation of EPR-detectable iron-nitrosyl complexes in islets of Langerhans. Role of nitric oxide in interleukin1 beta-induced inhibition of insulin secretion. Joumal of biological chemistry 266:21351-21354, 1991
32. Welsh N, Eizirik DL, Bendtzen K, Sandler S: Interleukin-1 beta-induced nitric oxide production in isolated rat pancreatic islets requires gene transcription and may lead to inhibition of the Krebs cycle enzyme aconitase. Endocrinology 129:3167-3173.1991
33. Biamonto G, Ruggiu M, Saccone S, Dellavalle G, Rive S: 2 homologous genes, originated by duplication, encode the human hnmp protein-a2 and protein-al. Nucleic Acids Research 22:1996-2002,1994
34. Dater KV, Dreyfuss G, Swanson MS: The human hnRNP M proteins: identification of a methionine/arginine-rich repeat motif in ribonucleoproteins. Nucleic acids research 21:439-446,1993
35. Eizirik DL, Bjorklund A, Welsh N: Interieukin-1-induced expression of nitric oxide synthase in insulin-producing cells is preceded by c-fos induction and depends on gene transcription and protein synthesis. FEBS letters 311: 62-66,1993
36. Chen MC, Schuit F, Pipeleers DG, Eizirik DL: IL-1beta induces serine protease inhibitor 3 (SPI3) gene expression in rat pancreatic beta-cells. Detection by differential display of messenger RNA. Cytokine 11:856-862,1999
37. Spinas GA, Hansen BS, Linde S, Kastem W, Molvig J, Mandrup-Poulsen T, Dinarello CA, Nielsen JH, NerupJ.: Interleukin 1 dose-dependently affects the biosynthesis of (pro)insulin in isolated rat islets of Langerhans. Diabetologia 30:474-480,1987
38. Riera M, Roher N, Miro F, Gil C, Trujillo R, Aguilera J, Plana M, Itade E: Association of protein kinase CK2 with eukalyotic translation initiation factor eIF-2 and with grp94/ndoplasmin. Molecular and Cellular Biochemistry 191:97-104,1999
39. Ramakrishnan M, Schonthal AH, LeeS: Endoplasmic reticulum stress-inducible protein GRP94 is associated with an Mg+-dependent serine kinase activity modulated by Ca2+ and GRP78/BiP. Journal of Cellular Physiology 170:115-129,1997
40. Hendershot I-M, Valentine VA, Lee AS, Morris SW, Shapiro DN: Localization of the gene encoding human BiP/GRP78, the endoplasmic reticulum cognate of the HSP70 family, to chromosome 9q34. Genomics 20:281-284,1994
41. Oliver JD, van-der W, F.J., Buileld NJ, High S: Interaction of the thiol-dependent reductase ERp57 with nascent glycoproteins. Science 275:86-88,1997
42. Nakamura M, Yamanobe T, Suyemitsu T, Komukai M, Kan R, Okinaga S, Aral K: A new membrane-associated Ca(2+)-binding protein of rat spermatogenic cells: its purification and characterization. Biochemical and biophysical research communications 176:1358-1364,1991
43. Bellmann K, Jaattela M, Wissing D, Burkad V, Kolb H: Heat shock protein hsp70 overexpression confers resistance against nitric oxide. FEBS letters 391:185-188,1996
44. Scarim AL, Heitmeier MR. Corbett JA: Heat shock inhibits cytokine-induced nitric oxide synthase expression by rat and human islets. Endocrinology 139:5050-5057,1998
45. Ankarcrona M, Dypbukt JM, Brune S, Nicotera P: Interleukin-1 beta-induced nitric oxide production activates apoptosis in pancreatic RINm5F cells. Experimental cell research 213:172-177, 1994
46, Vassiliadis S, Dragiotis V, Protopapadakis E, Athanassakis I, Mitlianga P, Konidads K, Papadopoulos GK: The destructive action of IL-lalpha and IL-1beta in IDDM is a multistage process: evidence and confirmation by apoptotic studies, induction of intermediates and electron microscopy. Mediators of inflammation 8:85-91,1999
47, Kaneto H, Fujii J, Seo HG, Suzuki K, Matsuoka T, Nakamura M, Tatsumi H, Yamasaki Y, Kamada T, Taniguchi N: Apoptotic cell-death triggered by nitric-oxide in pancreatic beta-cells. Diabetes 44:733-738,1995
48. Lotz MM, Andrews CW, Korzelius CA, Lee EC, Steele GD, Clarke A, Mercurio AM: Decreased expression of Mac-2 (carbohydrate binding protein 35) and loss of its nuclear localization are associated with the neoplastic progression of colon carcinoma. Proceedings of the National Academy of Sciences of the United States of America 90:3466-3470,1993
49. Hsu DK, Dowling CA, Jeng KCG, Chen JT, Yang RY, Liu FT: Galectin-3 expression is induced in cirrhotic liver and hepatocellular carcinoma. International Joumal of Cancer 81:519-526,1999
50. Karisen AE, Andersen HU, Mose Larsen P, Fey SJ, Larsen M, Pociot F, Whitmore T, Nielsen K, Nerup J: Galectin-3, a lectin involved in cytokine-mediated beta-cell destruction and IDDM? Diabetologia 40:A35,1997
51. - Sudo K, Takahashi E, Nakamura Y: Isolation and mapping of the human EIFAZ gene homologous to the murine protein synthesis initiation factor 4A-II gene Eif4a2. Cytogenetics and cell genetics 71:385-388,1995
52. Minturn JE, Fryer HJ, Geschwind DH, Hockfield S: TOAD-64, a gene expressed early in neuronal differentiation in the rat, is related to unc-33, a C, elegans gene involved in axon outgrowth. Journal of neuroscience15:6757-6766,1995
53. Wagner L, Oliyamyk 0, Gartner W, Nowotny P, Groeger M, Kaserer K, Waldhausl W, Pasternack MS: Cloning and expression of secretagogin, a novel neuroendocrine- and pancreatic islet of Langerhans-specific Ca2+-binding protein. Journal of Biological Chemistry 275:24740-24751, 2000
54. Rabinovitch A, Suarez- Pinzon W, Strynadka K, Sooy K, Christakos S: Calbindin-D28k overexpression prevents cytokine-induced apoptosis in pancreatic islet beta-cells. Diabetes 48:A427-428,1999
55. Bums K, Duggan B, Atkinson EA, Famulski KS, Nemer M, Bleackley RC, Michalak M: Modulation of gene expression by calreticulin binding to the glucocorticoid receptor. Nature 367:476-480,1994
56. Dedhar S, Ronnie PS, Shago M, Hagesteijn CY, Yang H, Filmus J, Hawley RG, Bruchovsky N, Cheng H, Matusik RJ: Inhibition of nuclear hormone receptor activity by calreticulin. Nature 1367:480-483,1994
57. Shimizu S, Narita M, Tsujimoto Y: Bcl-2 family proteins regulate the release of apoptogenic cytochrome c by the mitochondrial channel VDAC. Nature 399:483-487, 1999
58, Tiedge M, Lortz S, Munday IR, Lenzen S: Protection against the co-operative toxicity of nitric oxide and oxygen free radicals by overexpression of antioxidant enzymes in bioengineered insulin producing RINm5F cells. Diabetologia 42:849-855,1999
59. - Goth L, Eaton JW: Hereditary catalase deficiencies and increased risk of diabetes. Lancet (North American Edition) 356:1820-1821, 2000
60. Meister A, Anderson ME: Glutathione. Annual review of biochemistry 52:711-760,1983
61. Uhlig S, Wendel A: The physiological consequences of glutathione variations. Life sciences 51:1083,1094,1992
62. Casanova ML, Bravo A, Ramirez A, Morreale-de E, G" Were F, Medino G, Vidal M, Jorcano JL: Exocrine pancreatic disorders in transgenic mice expressing human keratin 8. Journal of clinical investigation 103:1587-1595,1999
63. Selmin 0, Lucier GW, Gark GC, Tritscher AM, Vanden-Heuvel JP, Gastel JA, Walker NJ, Sutter TR, Bell DA: Isolation and characterization of a novel gene induced by 2,3,i,8-tetrachlorodibenzo-p-dioxin in rat liver. Carcinogenesis 17:2609-2615,1996
64. Oppermann UC, Salim S, Tjernberg L0, Terenius L, Jomvall H: Binding of amyloid beta-peptide to mitochondrial hydroxyacyl-CoA dehydrogenase (ERAB): regulation of an SDR enzyme activity with implications for apoptosis in Alzheimer's disease. FEBS letters 451:238-242,1999
65. Sandford GR, Ho K, Burns WH: Characterization of the major locus of immediate-early genes of rat cytomegalovirus. Journal of virology 67:4093-4103,1993
66. Lahm HW, Langen H: Mass spectrometry: A tool for the identification of proteins separated by gels. Electrophoresis 21:2105-2114, 2000
67. Rabinovitch A, Suarez-Pinzon WI., Sorensen 0, Bleackley RC: Inducible nitric oxide synthase (iNOS) in pancreatic islets of nonobese diabetic mice: identification of iNOS-expressing cells and relationships to cytokines expressed in the islets. Endocrinology 137:2093-2099,1996
68. Christensen U, Spanre T, Cooke A, Andersen H, Mandrup-Poulsen T, Nerup J: Syngeneic islet transplantation in prediabetic BB-DP rats-a synchronized model for studying beta-cell destruction during the development of IDDM. Autoimmunity 28:91-107,1998
69. - Sparre T, Christensen UB, Mose Larsen P, Fey SJ, Karisen AE, Pociot F, Gotfredsen C, Richter B, Mandrup-Poulsen T, Nerup J: Dynamic changes in protein expression in syngeneic islet transplants during IDDM development in DP-BB rats. Diabetologia 41:A157,1998

## Claims

1. A method for diagnosing diabetes in a human, wherein the method comprises establishing in a biological sample from the human an increased expression of marker protein Zink Finger protein 43 (P28160).

2. A method according to claim 1, wherein the biological sample is selected from the group consisting of urine, blood, lymphatic fluids, and tissue.

3. A method according to claim 2, wherein the tissue is pancreatic tissue.

4. A method for diagnosing in a human the predisposition for diabetes, the method comprising establishing an increased expression in a biological sample from the human of marker protein Zink Finger protein 43 (P28160).

5. A test kit for diagnosing diabetes or a genetic predisposition for diabetes in a mammal, comprising:
a) a binding means which specifically binds to Zink Finger protein 43 (P28160) or an antibody for Zink Finger protein 43 (P28160), a nucleic acid fragment capable of binding to Zink Finger protein 43 (P28160), or a compound capable of binding to Zink Finger protein 43 (P28160),
b) means for detecting binding, if any, or the level of binding, of the binding means to Zink Finger protein 43 (P28160), and
c) means for correlating whether binding, if any, or the level of binding, to said binding means is indicative of the individual mammal having a significantly higher likelihood of having diabetes or a genetic predisposition for having diabetes.
